(19) Europäisches Patentamt
European Patent Office
Office européen des brevets

(11) Veröffentlichungsnummer: **0 007 471**
**B1**

(12) # EUROPÄISCHE PATENTSCHRIFT

(45) Veröffentlichungstag der Patentschrift: **04.11.81**

(21) Anmeldenummer: **79102225.4**

(22) Anmeldetag: **02.07.79**

(51) Int. Cl.³: **C 07 C 79/35,** A 01 N 33/22, A 01 N 37/34, A 01 N 37/40, C 07 C 103/26, C 07 C 121/75, C 07 C 153/05

(54) **Herbizid aktive 3,4-Dinitro-diphenyläther, ihre Herstellung, herbizide Mittel und ihre Verwendung.**

(30) Priorität: **04.07.78 CH 7301/78**

(43) Veröffentlichungstag der Anmeldung: **06.02.80 Patentblatt 80/3**

(45) Bekanntmachung des Hinweises auf die Patenterteilung: **04.11.81 Patentblatt 81/44**

(84) Benannte Vertragsstaaten: **CH DE FR GB IT NL**

(56) Entgegenhaltungen:
**DE-A-2 336 090**
**DE-A1-2 829 169**
**FR-A-1 502 538**
**DD-A-106 542**
**US-A-4 086 364**

(73) Patentinhaber: **CIBA-GEIGY AG, Patentabteilung Postfach, CH-4002 Basel (CH)**

(72) Erfinder: **Dürr, Dieter, Dr., Brändelistalweg 16, CH-4103 Bottmingen (CH)**
Erfinder: **Böhner, Beat, Dr., Hügelweg 3, CH-4102 Binningen (CH)**
Erfinder: **Pissiotas, Georg, Dr., Breslauerstrasse 8, D-7850 Lörrach (DE)**
Erfinder: **Rohr, Otto, Dr., Kilbertweg 19, CH-4106 Therwil (CH)**

(74) Vertreter: **Zumstein sen., Fritz, Dr. et al, Bräuhausstrasse 4, D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Herbizid aktive 3,4-Dinitro-diphenyläther, ihre Herstellung, herbizide Mittel und ihre Verwendung

Die vorliegende Erfindung betrifft herbizid aktive 3,4-Dinitro-diphenyläther.

Nitrierte Diphenyläther mit herbizider Wirkung sind aus verschiedenen Publikationen bekannt geworden. Es sind ebenfalls herbizid aktive 2,4-Dinitro-diphenyläther bekannt geworden, siehe z. B. die japanischen Patent-Publikationen No. 8 278/65, 8 279/65, 29 039/65 sowie das französische Patent No. 1 502 538 oder das belgische Patent No. 628 133. Die herbizide Wirkung der dort beschriebenen 2,4-Dinitro-diphenyläther ist jedoch nicht immer befriedigend.

Eine ganze Reihe von Patenten umfaßt in ihrem Schutzumfang auch 3,4-Dinitro-diphenyläther, ohne daß solche auch tatsächlich hergestellt und auf herbizide Wirkung geprüft worden wären, vielleicht wegen ihrer auf synthetischem Weg nicht leichten Zugänglichkeit.

Die vorliegende Erfindung betrifft noch nicht beschriebene 3,4-Dinitro-diphenyläther. Es wurde überraschenderweise gefunden, daß diese 3,4-Dinitro-diphenyläther starke herbizide Eigenschaften aufweisen und dies bereits bei geringen Aufwandmengen und dadurch bekannten nitrierten Diphenyläthern an Wirkung überlegen sind.

Die neuen 3,4-Dinitro-diphenyläther entsprechend der Formel I

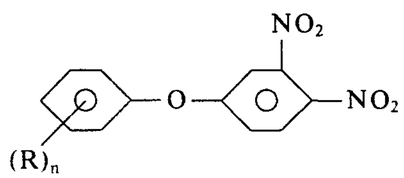

(I)

worin

R Halogen, Trifluormethyl, Nitro, Cyano, Carbamoyl ($-CONH_2$) oder Thiocarbamoyl ($-CSNH_2$) und
n eine Zahl von 0 bis 3 bedeuten.

Bevorzugt sind diejenigen Verbindungen, welche der Formel

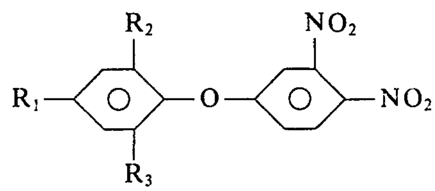

entsprechen, worin $R_1$, $R_2$ und $R_3$ Wasserstoff oder dasselbe wie R bedeuten.

Die Herstellung der erfindungsgemäßen 3,4-Dinitro-diphenyläther erfolgt in an sich bekannter Weise durch Umsetzen in Gegenwart einer Base als säurebindendes Mittel, vorzugsweise in einem inerten organischen Lösungsmittel, von meta-Nitrophenol mit einem in geeigneter Weise substituierten Halogenbenzol gemäß dem untenstehenden Schema

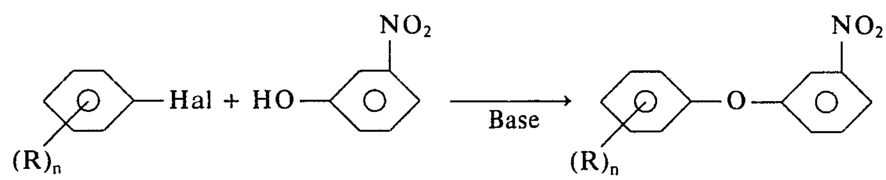


und Nitrierung in an sich bekannter Weise mit einem Nitriersäuregemisch des entstandenen 3-Nitrodiphenyläthers, wobei in den obigen Formeln »Hal« für ein Halogenatom steht, während R und n die unter Formel I gegebene Bedeutung haben.

Die genannte Umsetzung kann in An- oder Abwesenheit von gegenüber den Reaktionsteilnehmern inerten Lösungs- oder Verdünnungsmitteln durchgeführt werden, bevorzugt sind polare organische Lösungsmittel wie Methyläthylketon, Dimethylformamid, Dimethylsulfoxid. Die Reaktionstemperaturen liegen zwischen 0 und 200°, vorzugsweise 20 bis 100°C und die Reaktionsdauer beträgt je nach Ausgangsstoff, gewählter Umsetzungstemperatur und Lösungsmittel zwischen 1 Stunde und mehreren Tagen. Man arbeitet in der Regel bei Normaldruck. Als Basen (Kondensationsmittel) für die Umsetzung kommen die üblichen, wie z. B. KOH, $NaOCH_3$, $NaHCO_3$, $K_2CO_3$, Kalium-tert.butylat aber auch organische Basen wie Triäthylamin in Betracht.

Das als Ausgangsstoff benötigte meta-Nitrophenol ist bekannt, ebenso die für diese Umsetzung in

Frage kommenden, in geeigneter Weise substituierten Halogenbenzole.

Für die Einführung der para-ständigen Nitrogruppe behandelt man den 3-Nitro-diphenyläther nach bekannten Methoden mit Nitriersäuregemischen. Als solche kommen konzentrierte Schwefelsäure-, Salpetersäuregemische, konzentrierte Schwefelsäure und Alkalinitratsalze in Frage. Die Nitrierung verläuft exotherm und wird unter Normaldruck unter Kühlung oder höchstens bei Raumtemperatur vorgenommen.

Die nachfolgenden Beispiele veranschaulichen das erfindungsgemäße Verfahren für willkürlich ausgewählte 3,4-Dinitro-diphenyläther. Weitere in entsprechender Weise hergestellte Wirkstoffe sind in den anschließenden Tabellen aufgeführt. Temperaturangaben beziehen sich jeweils auf Celsius-Grade.

## Beispiel 1

3,4-Dinitro-2'-chlor-4'-trifluormethyl-diphenyläther

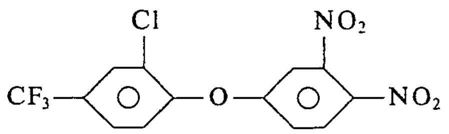

50 g 3-Nitro-2'-chlor-4'-trifluormethyl-diphenyläther werden in 200 ml konz. Schwefelsäure und 100 ml Äthylenchlorid vorgelegt. Unter gutem Rühren trägt man in kleinen Portionen insgesamt 16,5 g Kaliumnitrat bei 0° ein. Man entfernt das Kühlbad und läßt bei Raumtemperatur einige Zeit nachrühren. Zur Aufarbeitung gießt man das Reaktionsgemisch auf Eis, trennt die organische Schicht ab, wäscht mit Wasser neutral, dampft ein und trennt durch Destillation im Kugelrohrofen von etwas Ausgangsprodukt ab. Die Titelverbindung destilliert bei 180°/0,04 mb und schmilzt nach Kristallisation bei 81°.

## Beispiel 2

3,4-Dinitro-2'-chlor-4'-nitro-diphenyläther

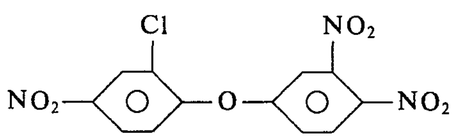

59 g 3-Nitro-2'-chlor-4'-nitro-diphenyläther werden in 175 ml konz. Schwefelsäure und 75 ml Methylenchlorid bei 15° vorgelegt. Eintropfen von 13 g rauchender Salpetersäure, gelöst in 20 g konz. Schwefelsäure.

Man läßt noch 10 Stunden bei Raumtemperatur nachrühren, um dann das Reaktionsgemisch auf Eis zu gießen. Der Niederschlag wird gesammelt, getrocknet und aus Chloroform/Hexan umkristallisiert. Man erhält 46 g der Titelverbindung. Smp. 129—130°.

In analoger Weise zu diesen Beispielen wurden folgende 3,4-Dinitro-diphenyläther hergestellt:

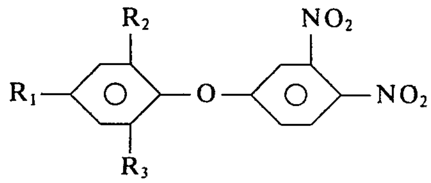

| No. | $R_1$ | $R_2$ | $R_3$ | Physikalische Konstante |
|---|---|---|---|---|
| 1 | $CF_3$ | Cl | H | Smp. 81° |
| 2 | $NO_2$ | Cl | H | Smp. 129–130° |
| 3 | Cl | $NO_2$ | H | Smp. 154° |
| 4 | $CF_3$ | $NO_2$ | H | Smp. 113–114° |
| 5 | $NO_2$ | $CF_3$ | H | Smp. 111° |
| 6 | $CF_3$ | CN | H | Smp. 108–110° |
| 7 | $CF_3$ | $CONH_2$ | H | Smp. 158–160° |
| 8 | $NO_2$ | $CONH_2$ | H | Smp. 206° |
| 9 | $CF_3$ | CL | Cl | |
| 10 | $NO_2$ | CN | H | |
| 11 | $CF_3$ | H | H | |
| 12 | $CF_3$ | $CF_3$ | H | |
| 13 | CN | $CF_3$ | H | |
| 14 | Cl | CN | H | |
| 15 | CN | Cl | H | |

Die neuen Wirkstoffe der Formel I sind stabile Verbindungen, welche in üblichen organischen Lösungsmitteln, wie Alkoholen, Äthern, Ketonen, Dimethylformamid, Dimethylsulfoxyd löslich sind. Sie können für sich allein, vorteilhafterweise aber zusammen mit geeigneten Trägerstoffen und/oder anderen Zuschlagstoffen als sogenannte Mittel verwendet werden.

Die Herstellung erfindungsgemäßer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

Feste Aufarbeitungsformen:
Stäubemittel, Streumittel, Granulate, Umhüllungsgranulate, Imprägniergranulate und Halogengranulate;
In Wasser dispergierbare Wirkstoffkonzentrate:
Spritzpulver (wettabele powder), Pasten, Emulsionen; Emulsionskonzentrate
Flüssige Aufarbeitungsformen:
Lösungen

Die Wirkstoffkonzentrationen betragen in den erfindungsgemäßen Mitteln 1 bis 80 Gewichtsprozent und können gegebenenfalls bei der Anwendung auch in geringen Konzentrationen wie etwa 0,05 bis 1% vorliegen.

Den beschriebenen erfindungsgemäßen Mitteln lassen sich andere biozide Wirkstoffe oder Mittel beimischen. So können die neuen Mittel außer den genannten Verbindungen der allgemeinen Formel I z. B. Insektizide, Fungizide, Bakterizide, Fungistatika, Bakteriostatika, Nematozide oder weitere Herbizide zur Verbreiterung des Wirkungsspektrums enthalten.

Die Herstellung erfindungsgemäßer Mittel sei durch Beispiele zur Herstellung fester und flüssiger Aufbereitungsformen näher erläutert.

## Granulat

Zur Herstellung eines 5%igen Granulates werden die folgenden Stoffe verwendet:

| | |
|---|---|
| 5 Teile | eines der Wirkstoffe der Formel I, |
| 0,25 Teile | Epichlorhydrin, |
| 0,25 Teile | Cetylpolyglykoläther, |
| 3,50 Teile | Polyäthylenglykol, |
| 91 Teile | Kaolin (Korngröße: 0,3–0,8 mm). |

Die Aktivsubstanz wird mit Epichlorhydrin vermischt und in 6 Teilen Aceton gelöst, hierauf wird Polyäthylenglykol und Cetylpolyglykoläther zugesetzt. Die so erhaltene Lösung wird auf Kaolin aufgesprüht und anschließend im Vacuum verdampft.

Spritzpulver

Zur Herstellung eines a) 70%igen und b) 10%igen Spritzpulvers werden folgende Bestandteile verwendet:

a) 70 Teile eines erfindungsgemäßen 3,4-Dinitro-diphenyläthers
5 Teile Natriumdibutylnaphthylsulfonat,
3 Teile Naphthalinsulfonsäuren-Phenolsulfonsäuren-Formaldehyd-Kondensat 3 : 2 : 1,
10 Teile Kaolin,
12 Teile Champagne-Kreide;

b) 10 Teile eines erfindungsgemäßen 3,4-Dinitro-diphenyläthers,
3 Teile Gemisch der Natriumsalze von gesättigten Fettalkoholsulfaten,
5 Teile Naphthalinsulfonsäuren-Formaldehyd-Kondensat
82 Teile Kaolin.

Der angegebene Wirkstoff wird auf die entsprechenden Trägerstoffe (Kaolin und Kreide) aufgezogen und anschließend vermischt und vermahlen. Man erhält Spritzpulver von vorzüglicher Benetzbarkeit und Schwebefähigkeit. Aus solchen Spritzpulvern können durch Verdünnung mit Wasser Suspensionen von 0,1—8% Wirkstoff erhalten werden, die sich zur Unkrautbekämpfung in Pflanzenkulturen eignen.

Paste

Zur Herstellung einer 45%igen Paste werden folgende Stoffe verwendet:

45 Teile 3,4-Dinitro-2′-chlor-4′-nitro-diphenyläther oder ein anderer Wirkstoff der Formel I,
5 Teile Natriumaluminiumsilikat,
14 Teile Cetylpolyglykoläther mit 8 Mol Äthylenoxid,
1 Teil Oleylpolyglykoläther mit 5 Mol Äthylenoxid,
2 Teile Spindelöl,
10 Teile Polyäthylenglykol,
23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 25%igen Emulsionskonzentrates werden

25 Teile eines Wirkstoffes der Formel I,
5 Teile einer Mischung von Nonylphenolpolyoxyäthylen oder Calciumdodecylbenzolsulfat,
15 Teile Cyclohexanon,
55 Teile Xylol

miteinander vermischt. Diese Konzentration kann mit Wasser zu Emulsionen auf geeignete Konzentrationen von z. B. 0,1 bis 10% verdünnt werden. Solche Emulsionen eignen sich zur Bekämpfung von Unkräutern in Kulturpflanzungen.

Zum Nachweis der Brauchbarkeit als Herbizide (pre- und post-emergent) und zum Beweis der Überlegenheit gegenüber bekannten Wirkstoffen ähnlicher Struktur dienen folgende Testmethoden:

Pre-emergente Herbizid-Wirkung (Keimhemmung)

Im Gewächshaus wird unmittelbar nach der Einsaat der Versuchspflanzen in Saatschalen die Erdoberfläche mit einer wässerigen Dispersion der Wirkstoffe, erhalten aus einem 25%igen Emulsionskonzentrat resp. aus einem 25%igen Spritzpulver mit Wirkstoffen, die wegen ungenügender Löslichkeit nicht als Emulsionskonzentrat hergestellt werden können, behandelt. Es wurden vier verschiedene Konzentrationsreihen angewendet, entsprechend 4, 2, 1 und 0,5 kg Wirksubstanz pro Hektar. Die Saatschalen werden im Gewächshaus bei 22—25°C und 50—70% rel. Luftfeuchtigkeit gehalten und der Versuch nach 3 Wochen ausgewertet und die Resultate nach folgender Notenskala bonitiert:

1 = Pflanzen nicht gekeimt oder total abgestorben
2—3 = sehr starke Wirkung
4—6 = mittlere Wirkung
7—8 = geringe Wirkung
9 = keine Wirkung (wie unbehandelte Kontrolle)
— = Pflanze in entsprechender Wirkstoffkonzentration nicht geprüft.

In diesem Test wurde der erfindungsgemäße 3,4-Dinitro-2'-chlor-4'-trifluormethyl-diphenyläther vom Beispiel 1 (1) mit dem strukturähnlichen 2,3'-Dinitro-4'-methyl-4-trifluormethyl-diphenyläther gemäß der belgischen Patentschrift No. 628 133 (A), sowie dem 2,4-Dinitro-2',4'-dichlor-diphenyläther (B) gemäß der japanischen Patentpublikation JA 29 039/65 verglichen.

| Verbindung | No. 1 | | A | | B | |
|---|---|---|---|---|---|---|
| Aufwandmenge kg/ha | 16 | 4 | 16 | 4 | 16 | 4 |
| Pflanze | | | | | | |
| avena fatua | 3 | 5 | 8 | 9 | 9 | 9 |
| setaria italica | — | 1 | — | 9 | 9 | 9 |
| sinapis alba | 2 | 4 | 8 | 9 | 9 | 9 |
| stellaria media | — | 5 | — | 9 | 9 | 9 |

## Post-emergente Herbidzid-Wirkung (Kontaktherbizid)

Eine größere Anzahl (mindestens 7) Unkräuter und Kulturpflanzen, sowohl monocotyle wie dicotyle, wurden nach dem Auflaufen (im 4- bis 6-Blattstadium) mit einer wässerigen Wirkstoffdispersion in Dosierungen von 0,06; 0,125; 0,25; 0,5 kg Wirksubstanz pro Hektar auf die Pflanze gespritzt und diese bei 24°—26°C und 45—60% rel. Luftfeuchtigkeit gehalten. Mindestens 15 Tage nach Behandlung wird der Versuch ausgewertet und das Ergebnis wie im pre-emergent-Versuch nach derselben Notenskala bonitiert.

Der Vergleich des erfindungsgemäßen 3,4-Dinitro-diphenyläthers No. 1 mit dem strukturähnlichen 2,3'-Dinitro-diphenyläther (A) gemäß BE-PS 628 133 ergibt folgendes Resultat:

| Verbindung | No. 1 | A |
|---|---|---|
| Aufwandmenge kg/ha | 4 | 4 |
| Pflanze | | |
| avena fatura | 1 | 7 |
| setaria italica | 1 | 7 |
| lolium perenne | 1 | 7 |
| sinapis alba | 1 | 7 |
| stellaria media | 1 | 9 |
| solanum nigrum | 1 | 4 |
| phaseolus vulgare | 1 | 7 |

Der erfindungsgemäße 3,4-Dinitro-diphenyläther zeigte in diesen Tests überraschenderweise weit bessere herbizide Wirkung als der strukturähnliche 2,3'-Dinitro-diphenyläther.

## Selektive herbizide Wirkung auf Reis im Nachlaufverfahren

Reispflänzchen, welche 25 Tage alt sind, werden im Gewächshaus in große rechteckige tiefe Schalen verpflanzt. Zwischen die Reihen der Reispflanzen werden dann Samen der in Reiskulturen vorkommenden Unkräuter Echinochloa crus galli, Cyperus difformis, Ammania indica und Rotala indica gesät. Die Schalen werden gut bewässert und bei einer Temperatur von ca. 25° und hoher Luftfeuchtigkeit gehalten. Nach 12 Tagen, wenn die Unkräuter aufgelaufen sind und das 2—3-Blattstadium erreicht haben, wird die Erde in der Schale mit einer 2,5 cm hohen Schicht Wasser bedeckt. Der Wirkstoff wird dann als Emulsionskonzentrat mittels einer Pipette zwischen die Pflanzenreihen appliziert, wobei man das Emulsionskonzentrat so verdünnt und aufträgt, daß es einer Applikationsmenge im Feld von 2 und 1 kg Wirkstoff entspricht. Bei Applikation der Verbindungen No. 1 und 2 wurde der Reis im Gegensatz zu den Unkräutern nicht geschädigt.

**Patentansprüche**

1. 3,4-Dinitro-diphenyläther der Formel I

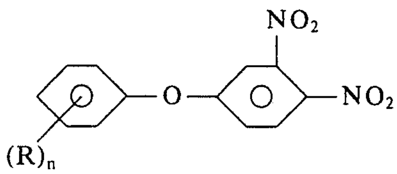

(I)

worin

R Halogen, Trifluormethyl, Nitro, Cyano, Carbamoyl ($-CONH_2$) oder Thiocarbamoyl ($-CSNH_2$) und
n eine Zahl 0 bis 3 bedeutet.

2. 3,4-Dinitro-diphenyläther gemäß Anspruch 1 der Formel

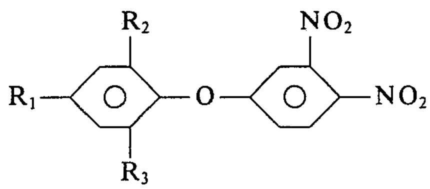

worin $R_1$, $R_2$ und $R_3$ Wasserstoff oder dasselbe wie R bedeuten.

3. 3,4-Dinitro-2'-chlor-4'-trifluormethyl-diphenyläther.

4. Verfahren zur Herstellung der 3,4-Dinitro-diphenyläther der Formel I gemäß Anspruch 1, dadurch gekennzeichnet, daß man in an sich bekannter Weise meta-Nitrophenol und ein Halogenbenzol der Formel

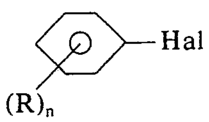

in Gegenwart einer Base als säurebindendes Mittel umsetzt, und den entstandenen 3-Nitro-diphenyläther der Formel

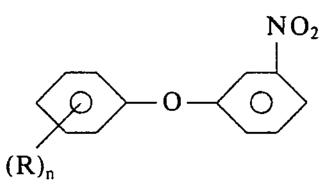

mit einem Nitriersäuregemisch nitriert, wobei in den obigen Formeln R und n die unter Formel I, Anspruch 1 gegebene Bedeutung haben und »Hal« für ein Halogenatom steht.

5. Herbizides Mittel, dadurch gekennzeichnet, daß es als Wirkstoff mindestens einen 3,4-Dinitro-diphenyläther gemäß Anspruch 1 enthält.

6. Verwendung der 3,4-Dinitro-diphenyläther gemäß Anspruch 1 oder sie enthaltender Mittel zur Unkrautbekämpfung.

7. Verwendung der 3,4-Dinitro-diphenyläther gemäß Anspruch 1 zur selektiven Bekämpfung von Unkräutern in Reiskulturen.

**Claims**

1. A 3,4-dinitro-diphenyl ether of the formula

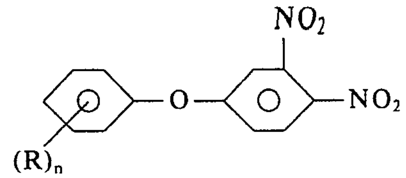

(I)

wherein R is halogen, trifluoromethyl, nitro, cyano, carbamoyl ($-CONH_2$) or thiocarbamoyl ($-CSNH_2$), and n is an integer from 0 to 3.

2. A 3,4-dinitro-diphenyl ether according to claim 1 of the formula

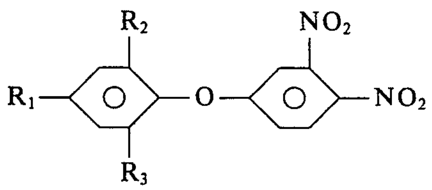

wherein $R_1$, $R_2$ and $R_3$ are hydrogen or have the same meaning as R.

3. 3,4-Dinitro-2′-chloro-4′-trifluoromethyldiphenyl ether.

4. A process for the manufacture of a 3,4-dinitrodiphenyl ether of the formula I according to claim 1, which process comprises reacting, in a manner known per se, meta-nitrophenol and a halobenzene of the formula

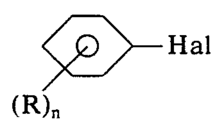

in the presence of a base as acid acceptor, and nitrating the resultant 3-nitrodiphenyl ether of the formula

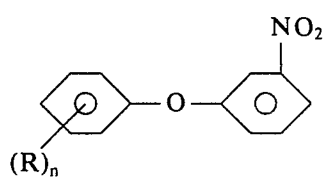

with a nitrating acid mixture, in which formulae above R and n are as defined for formula I in claim 1 and Hal is a halogen atom.

5. A herbicidal composition which contains, as active ingredient, at least one 3,4-dinitro-diphenyl ether according to claim 1.

6. A method of controlling weeds which comprises the use of a 3,4-dinitro-diphenyl ether according to claim 1 or of a composition containing such a compound.

7. A method of selectively controlling weeds in rice crops which comprises the use of a 3,4-dinitro-diphenyl ether according to claim 1.

**Revendications**

1. 3,4-dinitro-diphényléther de formule I

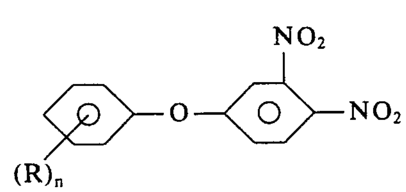

(I)

où R représente un halogène, un trifluorométhyle, un nitro, un cyano, un carbamoyl ($-CONH_2$) ou un thiocarbamoyle ($-CSNH_2$) et n un nombre allant de 0 à 3.

2. 3,4-dinitro-diphényléther selon la revendication 1 de formule:

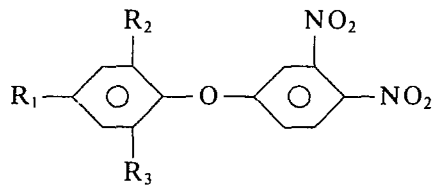

où $R_1$, $R_2$ et $R_3$ représentent un hydrogène ou ont la même signification que R.

3. 3,4-dinitro-2'-chloro-4'-trifluorométhyl-diphényléther.

4. Procédé de préparation des 3,4-dinitro-diphényléthers de formule I selon la revendication 1, caractérisé en ce qu'on fait réagir de façon classique du méta-nitrophénol et un benzène halogéné de formule:

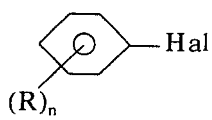

en présence d'une base sous forme de liant pour l'acide, et en ce qu'on nitre le 3-nitro-diphényléther de formule:

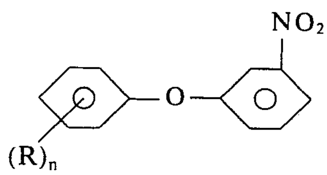

apparu, avec un mélange sulfonitrique, R et n ayant dans les formules ci-dessus la signification donnée pour la formule I de la revendication 1 et »Hal« représentant un atome d'halogène.

5. Agent herbicide, caractérisé en ce qu'il contient comme matière active au moins un 3,4-dinitro-diphényléther selon la revendication 1.

6. Application des 3,4-dinitro-diphényléthers selon la revendication 1 ou des agents qui les contiennent à la lutte contre les mauvaises herbes.

7. Application des 3,4-dinitro-diphényléthers selon la revendication 1 à la lutte sélective contre les mauvaise herbes dans les cultures de riz.